# FASCICULE DE BREVET EUROPEEN

(11) **EP 4 347 050 B1**
(45) Date de publication et mention de la délivrance du brevet: **04.03.2026**
(21) Numéro de dépôt: 22728294.4
(22) Date de dépôt: 25.05.2022
(51) Int. Cl.: A61Q 19/00, A61K 8/9789

(54) **MÉTHODE DE TRAITEMENT COSMÉTIQUE PAR ILLUMINATION ET APPLICATION COMBINÉE D'UNE COMPOSITION COMPRENANT UN EXTRAIT D'EPILOBIUM, ET DISPOSITIF ASSOCIÉ**
KOSMETISCHES BEHANDLUNGSVERFAHREN DURCH KOMBINIERTE BELEUCHTUNG UND ANWENDUNG EINER ZUSAMMENSETZUNG MIT EINEM EPILOBIUM-EXTRAKT UND ZUGEHÖRIGE VORRICHTUNG
METHOD FOR COSMETIC TREATMENT BY COMBINED ILLUMINATION AND APPLICATION OF A COMPOSITION COMPRISING AN EPILOBIUM EXTRACT, AND ASSOCIATED DEVICE

(30) Priorité: 26.05.2021 FR 2105450
(43) Date de publication de la demande: 10.04.2024
(73) Titulaire: Lightinderm, 75017 Paris (FR)
(72) Inventeur: DECLERCQ, Lieve, 9070 DESTELBERGEN (BE); GUERMONPREZ, CYPRIEN, 75011 Paris (FR)
(74) Mandataire: Delaveau, Sophie
(86) Numéro de dépôt international: PCT/IB2022/054900
(87) Numéro de publication internationale: WO 2022/249096

(56) Documents cités:
- US-A1- 2016 089 310
- US-A1- 2018 071 547
- DATABASE GNPD [online] MINTEL; 28 March 2019 (2019-03-28), ANONYMOUS: "Matte Moisturizing Fluid", XP055886658, retrieved from https://www.gnpd.com/sinatra/recordpage/6433145/ Database accession no. 6433145

## Description

### DOMAINE TECHNIQUE

L'invention s'inscrit dans le domaine des traitements cosmétiques non thérapeutiques par illumination de la peau. L'invention s'inscrit en outre dans le domaine des traitements cosmétiques par application d'une composition comprenant au moins un actif.

L'invention concerne plus particulièrement un traitement non thérapeutique destiné à limiter les désordres cutanés liés à la peau dite à tendance grasse.

L'invention porte en outre sur un dispositif de mise en oeuvre d'un tel procédé.

### ART ANTERIEUR

La peau grasse ou à tendance grasse se caractérise par une sécrétion importante de sébum. Le sébum est principalement constitué de lipides, essentiellement de triglycérides, de cires (esters d'acides gras) et d'alcools. Le sébum est sécrété dans les glandes sébacées situées sous la surface de la peau et le plus souvent reliées à un follicule pileux. En périphérie des glandes sébacées, des cellules différentiées, les sébocytes, synthétisent différents corps gras dont le sébum, et libèrent leur contenu dans la glande sébacée.

Une sécrétion importante de sébum confère à la peau un aspect luisant. Souvent également, la peau présente des orifices folliculaires dilatés. Des complications inflammatoires peuvent également être observées sur des peaux grasses, causées par différents facteurs, notamment la prolifération de bactéries.

Il existe des traitements destinés à réduire les effets inesthétiques et inflammatoires des peaux à tendance grasse. Mais le plus souvent, ces traitements sont soit peu efficaces, soit réalisés à partir de produits de synthèse pouvant engendrer d'autres effets indésirables sur la peau, par exemple un dessèchement accru.

US2016/089310 A1 divulgue une composition active comprenant un extrait d'*Epilobium fleischeri* en tant que principe actif pour controller le sébum.

L'invention a pour objet de proposer un traitement efficace contre de tels désordres cutanés, notamment pour réduire la production de sébum et matifier la peau.

L'invention a également pour objet de proposer un traitement qui permet en outre de corriger les imperfections et de resserrer les pores.

L'invention a enfin pour objet de proposer un traitement qui soit à la fois efficace contre les désordres cutanés liés aux peaux à tendance grasse, et qui contribue également à l'amélioration générale de l'état de la peau.

À cet effet, l'invention vise un procédé de traitement cosmétique non thérapeutique comprenant au moins les étapes de :
- application sur la peau d'un sujet d'une composition active comprenant au moins un extrait d*'Epilobium fleischeri,* et
- illumination de la peau par au moins une source lumineuse émettant à une longueur d'onde comprise entre 490 et 550 nanomètres, de préférence de 520 nanomètres,
l'illumination étant réalisée avant, simultanément ou après l'application de la composition.

Le procédé de l'invention peut également comporter les caractéristiques optionnelles suivantes considérées isolément ou selon toutes les combinaisons techniques possibles :
- la composition active comprend en outre de l'acide salicylique.
- la composition active comprend en outre de l'extrait d'artichaut.
- la composition active comprend en outre de la niacinamide, et l'illumination de la peau est en outre réalisée par une source lumineuse émettant à une longueur d'onde comprise entre 620 et 690 nanomètres, de préférence de 660 nanomètres.
- l'illumination de la peau est en outre réalisée par une source lumineuse émettant à une longueur d'onde comprise entre 410 et 470 nanomètres, de préférence entre 440 et 450 nanomètres.
- le pourcentage massique en extrait d'*Epilobium* dans la composition est compris entre 0,1 et 10%.

L'invention porte en outre sur un dispositif de traitement cosmétique non thérapeutique comprenant au moins des moyens d'application sur la peau d'un sujet d'une composition active comprenant au moins un extrait d'*Epilobium fleischeri,* laquelle composition active est contenue dans les moyens d'application, et des moyens d'illumination de la peau par au moins une source lumineuse émettant à une longueur d'onde comprise entre 490 et 550 nanomètres, de préférence de 520 nanomètres,
les moyens d'illumination et d'application de la composition active étant configurés pour que l'illumination puisse être réalisée avant, simultanément ou après l'application de la composition.

Avantageusement, la composition active comprend en outre de la niacinamide, et les moyens d'illumination de la peau comportent en outre une source lumineuse émettant à une longueur d'onde comprise entre 620 et 690 nanomètres, de préférence de 660 nanomètres.

Plus avantageusement encore, la composition active comprend en outre de l'acide salicylique et de l'extrait d'artichaut, et les moyens d'illumination de la peau comportent en outre une source lumineuse émettant à une longueur d'onde comprise entre 410 et 470 nanomètres, de préférence entre 440 et 450 nanomètres.

D'autres caractéristiques et avantages de l'invention ressortiront clairement de la description qui en est donnée ci-dessous, à titre indicatif et nullement limitatif, en référence aux figures annexées :

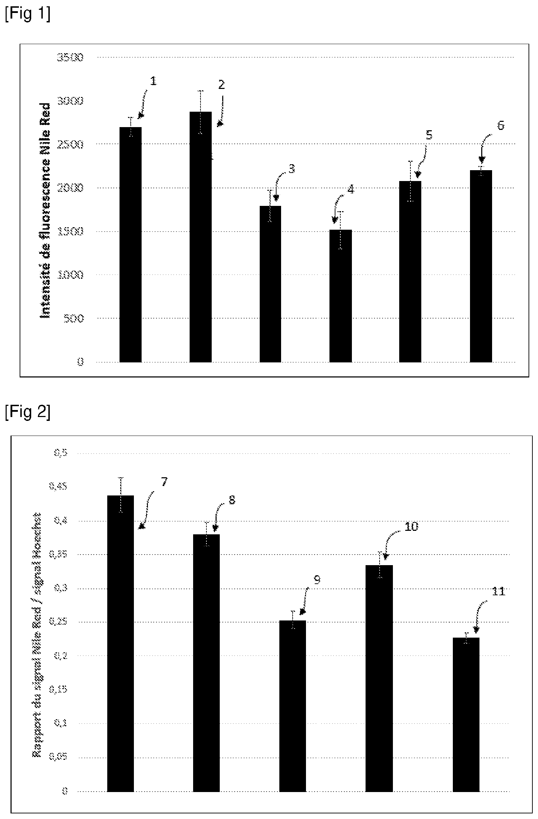

[Fig 1] La figure 1 est un histogramme illustrant la production de lipides dans des sébocytes à travers l'intensité du signal de fluorescence d'un marqueur, les sébocytes ayant été exposés à des UVB et traités par la combinaison de l'application d'une composition comprenant notamment un extrait d'*Epilobium fleischeri* et de différentes illuminations, dont une illumination émettant notamment à une longueur d'onde de 520 nanomètres ;
[Fig 2] La figure 2 est un histogramme illustrant la production de lipides dans des sébocytes ayant été exposés à des UVB, à travers le rapport entre le signal Nile Red et le signal Hoechst, traités par une illumination seule émettant à différentes longueurs d'ondes dont une longueur d'onde de 520 nanomètres, traités par l'application seule d'une composition comprenant notamment un extrait d'*Epilobium fleischeri,* et traités par la combinaison d'une illumination émettant à différentes longueurs d'ondes dont une longueur d'onde de 520 nanomètres, et d'une composition comprenant notamment un extrait d'*Epilobium fleischeri* ;

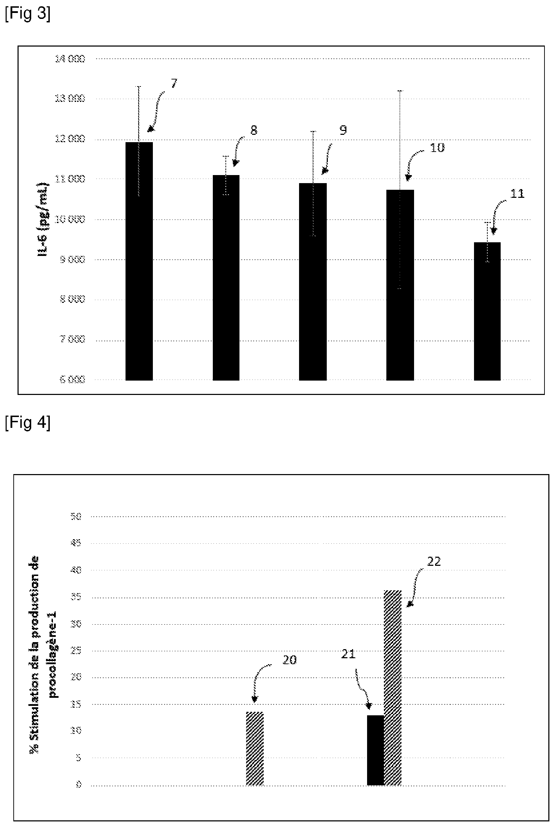

[Fig 3] La figure 3 est un histogramme illustrant la quantité d'interleukine 6 présente dans des sébocytes ayant été exposés à des UVB, traités par une illumination seule émettant à différentes longueurs d'ondes dont une longueur d'onde de 520 nanomètres, traités par l'application seule d'une composition comprenant notamment un extrait d'*Epilobium fleischeri* et traités par la combinaison d'une illumination émettant à différentes longueurs d'ondes dont une longueur d'onde de 520 nanomètres, et d'une composition comprenant notamment un extrait d'*Epilobium fleischeri* ;
[Fig 4] La figure 4 est un histogramme illustrant le pourcentage de stimulation de la production de procollagène-1 de cellules préalablement exposées à une combinaison d'UVA et d'UVB et traitées par une illumination seule à une longueur d'onde de 660 nanomètres, par l'application seule de niacinamide, et par un procédé de traitement avec une composition comportant de la niacinamide et une illumination discontinue à une longueur d'onde de 660 nanomètres, et par une illumination seule à une longueur d'onde de 660 nanomètres.

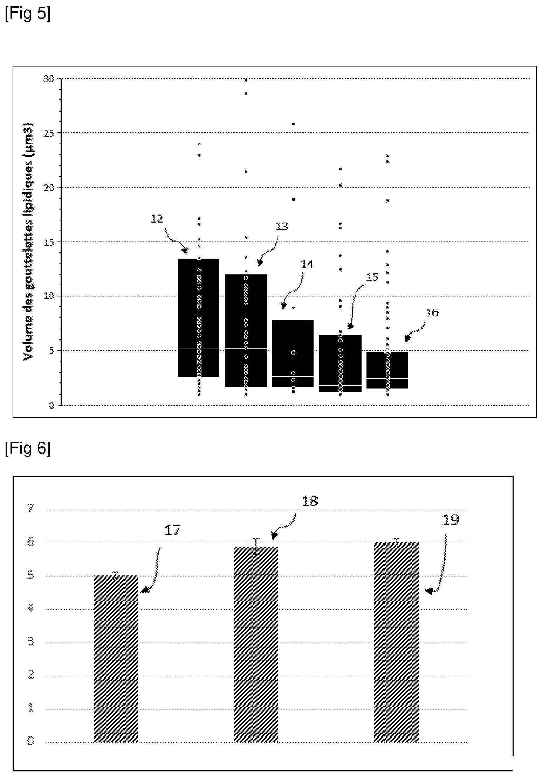

[Fig 5] La figure 5 est un histogramme illustrant la distribution du volume des gouttelettes lipidiques dans des glandes sébacées ayant été exposées à des UVB, traitées par une illumination seule émettant à différentes longueurs d'ondes dont une longueur d'onde de 520 nanomètres, traitées par une composition seule comprenant notamment un extrait d'*Epilobium fleischeri* et traités par la combinaison d'une illumination émettant à différentes longueurs d'ondes dont une longueur d'onde de 520 nanomètres, et d'une composition comprenant un extrait d'*Epilobium fleischeri* ;
[Fig 6] La figure 6 est un graphique illustrant l'évolution de l'hydratation de la peau du visage d'une femme (évaluation clinique) ayant bénéficié du procédé de traitement cosmétique de l'invention au premier jour de traitement, au 14^{ème} jour de traitement et au 28^{ème} jour de traitement ;

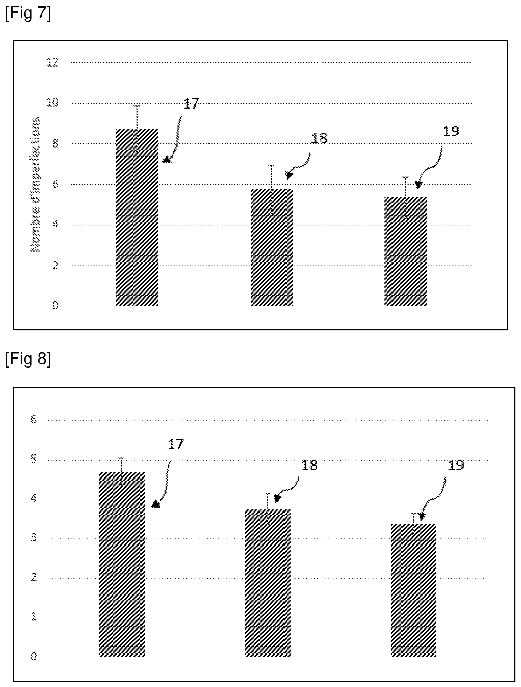

[Fig 7] La figure 7 est un graphique illustrant l'évolution du nombre d'imperfections sur la peau du visage d'une femme (évaluation clinique) ayant bénéficié du procédé de traitement cosmétique de l'invention au premier jour de traitement, au 14^{ème} jour de traitement et au 28^{ème} jour de traitement ;
[Fig 8] La figure 8 est un graphique illustrant l'évolution de la brillance de la peau du visage d'une femme (évaluation clinique) ayant bénéficié du procédé de traitement cosmétique de l'invention au premier jour de traitement, au 14^{ème} jour de traitement et au 28^{ème} jour de traitement ;

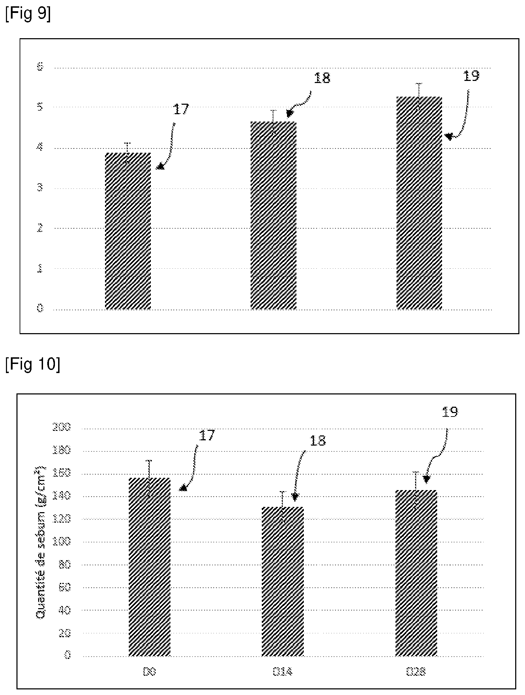

[Fig 9] La figure 9 est un graphique illustrant l'évolution de la douceur de la peau du visage d'une femme (évaluation clinique) ayant bénéficié du procédé de traitement cosmétique de l'invention au premier jour de traitement, au 14^{ème} jour de traitement et au 28^{ème} jour de traitement ;
[Fig 10] La figure 10 est un graphique illustrant l'évolution de la quantité de sébum sur le front du visage d'une femme (mesures quantitatives) ayant bénéficié du procédé de traitement cosmétique de l'invention au premier jour de traitement, au 14^{ème} jour de traitement et au 28^{ème} jour de traitement ;

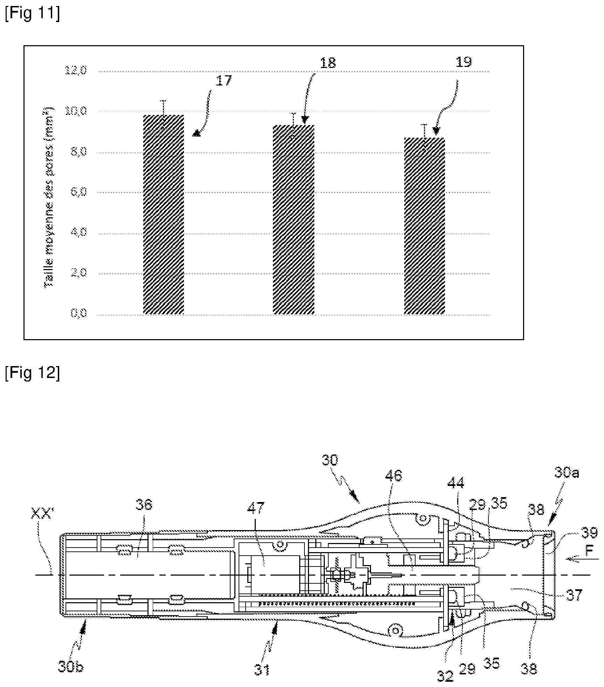

[Fig 11] La figure 11 est un graphique illustrant l'évolution de la taille moyenne des pores de la peau du visage d'une femme (mesures quantitatives) ayant bénéficié du procédé de traitement cosmétique de l'invention au premier jour de traitement, au 14^{ème} jour de traitement et au 28^{ème} jour de traitement ;
[Fig 12] La figure 12 est une représentation schématique en coupe du dispositif de l'invention dépourvu de sa capsule d'application de la composition,

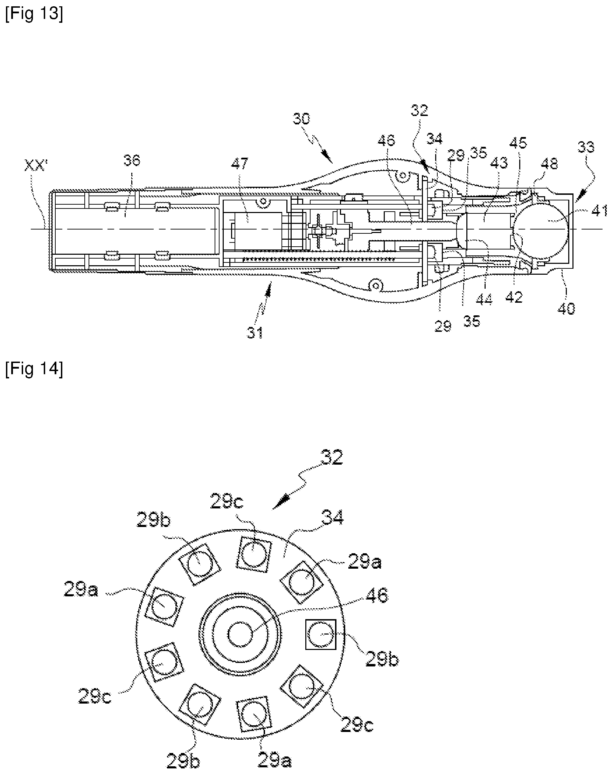

[Fig 13] La figure 13 est une représentation schématique en coupe du dispositif de l'invention illustré avec la capsule d'application de la composition, et
[Fig 14] La figure 14 est une représentation schématique de face d'un exemple de réalisation du dispositif électroluminescent du dispositif de l'invention des figures 12 et 13.

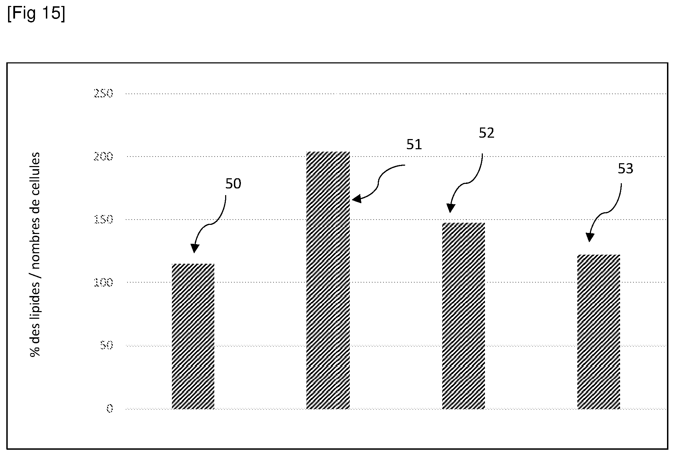

[Fig 15] La figure 15 est un histogramme illustrant le pourcentage de lipides par rapport au nombre de cellules (nucléi) pour des sébocytes respectivement non traités, supplémentés avec du LDS (Liquid Droplet Stimulator), supplémentés avec du LDS, exposés à des UVB et traités par un extrait d*'Epilobium fleischeri,* et supplémentés avec du LDS, exposés à des UVB et traités par un extrait d*'Epilobium fleischeri* et une illumination émettant à 520 nanomètres.

### DESCRIPTION DETAILLEE DE L'INVENTION

Le procédé de traitement cosmétique de l'invention est un procédé non thérapeutique qui s'applique exclusivement sur une peau dénuée de plaie ouverte et de toute autre pathologie. Le procédé de traitement cosmétique de l'invention s'applique à une peau dite « à tendance grasse », c'est-à-dire principalement soumise à une production excessive de sébum.

Le procédé de traitement cosmétique de l'invention comprend essentiellement une étape d'application sur la peau d'un sujet d'une composition comprenant au moins un extrait d*'Epilobium fleischeri* comme agent actif, et d'une illumination, de préférence simultanément, de cette même zone de la peau, par une source lumineuse émettant dans le spectre vert à une longueur d'onde d'environ 520nm, considérant une largeur de demi-bande de 30nm. Lorsque l'illumination est réalisée avant ou après l'application de la composition, on considère un temps maximum entre l'illumination et l'application de la niacinamide de 30 minutes, de préférence de 15 minutes.

Le procédé de traitement cosmétique de l'invention peut avantageusement inclure l'application concomitante sur la peau du sujet de niacinamide couplée à une illumination par une source lumineuse émettant dans le spectre rouge à une longueur d'onde d'environ 660nm, considérant une largeur de demi-bande de 30nm. L'application supplémentaire de niacinamide et d'une illumination par la lumière rouge permet d'apporter un soin de la peau en plus de la réduction des effets indésirables d'une peau à tendance grasse, comme il sera détaillé plus loin.

Le procédé de l'invention permet de réduire significativement la production de lipides par les sébocytes, ainsi que leur caractère inflammatoire. Le procédé de l'invention permet également de réduire significativement ex-vivo le volume de lipides produit par les glandes sébacées. Enfin, les tests cliniques qui seront détaillés plus loin mettent en évidence, en plus de la réduction de la quantité de sébum sur la peau et de l'aspect brillance associé, l'amélioration de l'hydratation de la peau, la réduction des imperfections et une amélioration générale de la qualité de la peau.

Des essais in-vitro de la production lipidique d'une culture de sébocytes soumise à différents traitements ont été menés. Trois jours après ensemencement, les sébocytes sont irradiés par des UVB, puis soumis au traitement. Le traitement est de nouveau appliqué au quatrième jour. Cinq jours après ensemencement, les sébocytes sont de nouveau irradiés par des UVB, puis soumis au traitement. Le traitement est de nouveau appliqué au sixième jour. Les cultures sont récupérées pour analyses au septième jour.

Le traitement consiste en l'application d'un sérum aqueux dont la composition est donnée dans le Tableau 1 ci-dessous.

**[Tableaux 1] : Composition du sérum**

| | |
|---|---|
| Alpaflor (extrait d'Epilobium Fleischeri) | 1,0% en poids |
| Niacinamide | 3,0% en poids |
| Extrait d'artichaut | 1,0% en poids |
| Acide salicylique | 0,5% en poids |
| Vitamine C | 0,2% en poids |

Le traitement consiste en outre en une illumination concomitante à l'application de la composition selon les quatre programmes d'illumination définis dans le Tableau 2 ci-dessous.

**[Tableaux 2] : Paramètres associés à chaque programme d'illumination testé**

| Programme | 1 | | 2 | |
|---|---|---|---|---|
| Longueur d'onde (nm) | Intensité (mA) | Irradiance (mW/cm2) | Intensité (mA) | Irradiance (mW/cm2) |
| 440 | 214 | 7,4 | 244 | 8,5 |
| 520 | - | - | 244 | 8,2 |
| 660 | 244 | 11 | 244 | 10,6 |
| 940 | 220 | 3,7 | - | - |

| Programme | 3 | | 4 | |
|---|---|---|---|---|
| Longueur d'onde (nm) | Intensité (mA) | Irradiance (mW/cm2) | Intensité (mA) | Irradiance (mW/cm2) |
| 440 | 117 | 4,1 | 489 | 16,9 |
| 520 | - | - | - | - |
| 660 | 98 | 4,2 | 489 | 21,2 |
| 940 | 64 | 1,1 | 489 | 8,2 |

On se reporte à la figure 1 illustrant sous forme d'histogramme la production de lipides dans des sébocytes à travers l'intensité du signal de fluorescence d'un marqueur. Sur cette figure 1, la référence 1 correspond à des sébocytes n'ayant subi ni irradiation, ni traitement, , la référence 2 correspond à une irradiation par des UVB non suivi d'un traitement, la référence 3 correspond à un traitement par application du sérum et une illumination selon le programme d'illumination 1 après irradiation par les UVB, la référence 4 correspond à un traitement par application du sérum et une illumination selon le programme d'illumination 2 après irradiation par les UVB, la référence 5 correspond à un traitement par application du sérum et une illumination selon le programme d'illumination 3 après irradiation par les UVB, et la référence 6 correspond à un traitement par application du sérum et une illumination selon le programme d'illumination 4 après irradiation par les UVB.

Les résultats présentés sur la figure 1 sont obtenus par prélèvement de la culture de sébocytes au septième jour après ensemencement, et marquage des cellules par un marqueur, le Nile Red. Ce marqueur est connu dans l'étude des structures lipidiques pour ses propriétés de fluorescence en milieu hydrophobe. Il forme ainsi une sonde hydrophobe qui s'insère dans les structures lipidiques. En intégrant le signal de fluorescence obtenu avec le Nile Red, on obtient alors une estimation de la quantité de lipides (sébum) dans les sébocytes.

On constate que si les quatre programmes d'illumination (Tableau 2) combinés à l'application du sérum (Tableau 1) permettent de réduire la quantité de sébum sécrété par les sébocytes, le programme d'illumination 2, qui est le seul à inclure une illumination à la lumière verte à une longueur d'onde de 520 nm, est celui qui conduit à la plus forte réduction de la quantité de sébum sécrétée. C'est ce programme d'illumination 2 qui est utilisé dans la suite des essais.

En référence aux figures 2 et 3, des essais ont de nouveau été conduits sur des cultures de sébocytes selon le même protocole qu'indiqué précédemment (ensemencement, irradiations aux UVB aux troisième et cinquième jours, application d'un traitement quotidien du troisième au sixième jour et récupération des cultures le septième jour) pour mettre en évidence la synergie entre le sérum comprenant l'extrait d*'Epilobium Fleischeri* et le programme d'illumination 2 comprenant une illumination à une longueur d'onde de 520nm.

Sur ces figures, la référence 7 correspond à des cultures de sébocytes de référence n'ayant subies ni irradiations aux UVB, ni traitement subséquent. La référence 8 correspond à des cultures de sébocytes qui ont été uniquement irradiées par des UVB. La référence 9 correspond à des cultures de sébocytes qui ont été irradiées par des UVB et traitées par la seule application du sérum. La référence 10 correspond à des cultures de sébocytes qui ont été irradiées par des UVB et traitées uniquement par illumination selon le programme 2. Et la référence 11 correspond à des cultures de sébocytes qui ont été irradiées par des UVB et traitées par l'application du sérum et une illumination conjointe selon le programme 2.

Les résultats présentés sur la figure 2 confirment les résultats présentés sur la figure 1 en ce que l'application conjointe de sérum comprenant l'extrait *d'Epilobium Fleischeri* et d'une illumination selon le programme d'illumination 2 comprenant une illumination à une longueur d'onde de 520nm, permettent de réduire substantiellement la sécrétion de sébum par les sébocytes. Cette évaluation est réalisée en procédant au rapport entre le signal Nile Red qui traduit le taux de lipides dans les sébocytes, et le signal Hoechst qui traduit la quantité de cellules.

On constate en outre que cette application conjointe du sérum et de l'illumination permet d'obtenir une meilleure réduction de la sécrétion de sébum que l'application seule du sérum ou que la seule l'illumination, mettant ainsi en évidence la synergie entre le sérum et l'illumination selon le programme d'illumination 2.

Les résultats présentés sur la figure 3 permettent également de mettre en évidence la synergie entre le sérum et l'illumination selon le programme d'illumination 2 pour ce qui est de la réduction du caractère inflammatoire. En effet, la figure 3 montre une réduction significative de la concentration en interleukine-6 relargué par les sébocytes pour le traitement du procédé de l'invention. Cette synergie est d'autant plus marquante que la seule application du sérum (référence 9) et la seule illumination par le programme d'illumination 2 (référence 10) conduisent à une faible réduction de la concentration en interleukine-6.

Les résultats présentés sur les figures 2 et 3 montrent ainsi respectivement une réduction de la production de lipides par les sébocytes et une réduction du relargage d'interleukine-6 et pour des cultures soumises au procédé de traitement de l'invention. Ces deux paramètres sont à considérer dans leur ensemble puisque dans le cas d'un tissu complet, il est connu que le caractère inflammatoire amplifie la production de sébum. Ainsi, le procédé de traitement de l'invention, agissant non seulement sur la production de sébum mais également sur le caractère inflammatoire, est substantiellement actif pour le traitement des peaux à tendance grasse.

Des essais ex-vivo sur la production lipidique de glandes sébacées soumises à différents traitements ont été menés. Un jour après ensemencement, les glandes sébacées sont irradiées par des UVB, puis soumises au traitement. Le traitement est appliqué quotidiennement jusqu'au quatrième jour. Au troisième jour, les glandes sébacées sont de nouveau irradiées aux UVB avant l'application du traitement quotidien. Les cultures sont récupérées pour analyses le cinquième jour. Les cultures récupérées font l'objet d'un marquage coloré des gouttelettes lipidiques. Une imagerie confocale permet de reconstruire en trois dimensions les glandes sébacées et de mesurer la quantité et la taille des gouttelettes lipidiques.

La figure 5 illustre les résultats obtenus en termes de distribution du volume des gouttelettes lipidiques contenues dans les glandes sébacées issues des cultures. La référence 12 correspond à une glande sébacée non traitée. La référence 13 correspond également à une glande sébacée non traitée. La référence 14 correspond à une glande sébacée ayant été uniquement traitée par l'application du sérum (Tableau 1). La référence 15 correspond à une glande sébacée traitée uniquement par une illumination selon le programme d'illumination 2 (Tableau 2). Et la référence 16 correspond à des glandes sébacées traitées par le procédé de l'invention, soit une combinaison de l'application du sérum et d'une illumination selon le programme d'illumination 2.

On constate que la plus forte réduction de la distribution du volume des gouttelettes lipidiques dans les glandes sébacées est obtenue pour un traitement selon l'invention combinant l'application du sérum et l'illumination par la lumière verte (référence 16). Ces résultats viennent une fois encore confirmer l'effet de synergie entre l'application du sérum et une illumination à une longueur d'onde de 520nm.

Ces résultats sont encore corroborés par la figure 15 qui illustre les résultats obtenus en termes de pourcentages de lipides par rapport au nombre de cellules pour des sébocytes respectivement non traités (référence 50), supplémentés en LDS (terme anglophone pour Lipid Droplet Stimulator - stimulateur lipidique) (référence 51), exposés à des UVB et traités par l'application seule d'un extrait d'*Epilobium fleischeri* (référence 52), et exposés à des UVB et traités par l'application conjointe d'un extrait d'*Epilobium fleischeri* et d'une illumination par la seule lumière verte à une longueur d'onde de 520 nm (référence 53). Pour ces deux derniers traitements, les sébocytes sont également supplémentés en LDS.

Les sébocytes faisant l'objet de ces résultats sont cultivés pendant 9 jours et le milieu de culture est changé chaque jour à partir du jour 5. C'est également au jour 5 que les sébocytes sont exposés une première fois aux UVB et traités par l'application de l'extrait d'*Epilobium fleischeri* et le cas échéant l'illumination par la lumière verte. Une seconde exposition aux UVB est réalisée au jour 7, tandis que le traitement par l'application de l'extrait d'*Epilobium fleischeri* et le cas échéant l'illumination par la lumière verte est appliqué aux jours 6, 7 et 8. Le jour 9 marque la fin du traitement et le début des analyses.

On constate par rapport aux sébocytes non traités (référence 50) et aux sébocytes non traités et supplémentés en LDS (référence 51) que si l'extrait d*'Epilobium fleischeri* seul (référence 52) induit une action non négligeable sur la production de lipides par les sébocytes, c'est bien l'application conjointe de l'extrait d'*Epilobium fleischeri* et de la lumière verte (référence 53) qui permet de retrouver un pourcentage de lipides produits par les sébocytes proche de celui des sébocytes non traités.

Des tests cliniques ont été menés sur 32 femmes de 20 à 40 ans présentant des phototypes de I à III. Pendant 28 jours, ces femmes ont reçu 6 jours sur 7 le traitement selon le procédé de l'invention. L'illumination correspond au programme 2 présenté sur le Tableau 2 et la composition est celle indiquée au Tableau 1. Le traitement est réalisé par application simultanée de la composition et de l'illumination selon le programme 2. Le traitement selon l'invention est effectué sur 12 zones du menton au front (6 zones par demi-face). Chaque zone est traitée pendant 15 secondes selon le programme 2; le traitement du visage est ainsi réalisé en 3 minutes.

Un spécialiste évalue cliniquement les effets du traitement au bout de 14 jours (référence 18 sur les figures 6 à 9) et 28 jours (référence 19 sur les figures 6 à 9), par comparaison au premier jour de traitement (référence 17 sur les figures 6 à 9), sur l'hydratation de la peau, sa brillance, le nombre d'imperfections, et le caractère lisse de la peau. Pour l'hydratation de la peau, la brillance et le caractère lisse, les résultats sont issus d'une évaluation clinique par scoring réalisée par un spécialiste. En référence à la figure 10, la quantité de sébum sur la peau est mesurée par un sébumètre également au bout de 14 jours (référence 18) et 28 jours (référence 19), par comparaison au premier jour de traitement (référence 17). Enfin, en référence à la figure 11, la taille moyenne des pores est évaluée par analyse d'images à haute définition selon le même protocole.

On constate que pour tous les paramètres testés qu'ils soient évalués cliniquement ou mesurés, le procédé de l'invention améliore la qualité de la peau tant sur son hydratation, la réduction de ses imperfections, la réduction de la brillance mais aussi sa douceur.

Le procédé de l'invention met en jeu plusieurs couples agent actif/ longueur d'onde. Outre la combinaison de l'extrait d'*Epilobium fleischeri* et d'une illumination à la lumière verte, le traitement comporte également une combinaison de niacinamide et de lumière rouge. En référence à la figure 4, on mesure le pourcentage de stimulation de la production de procollagène-1 de cellules préalablement exposées à une combinaison d'UVA et d'UVB et traitées par une illumination seule à une longueur d'onde de 660 nanomètres, par l'application seule de niacinamide, et par un procédé de traitement avec une composition comportant de la niacinamide et une illumination discontinue à une longueur d'onde de 660 nanomètres. La référence 20 correspond au traitement par la niacinamide uniquement, la référence 21 correspond à un traitement par une illumination à 660 nm seule, et la référence 22 correspond à un traitement combinant les effets de la niacinamide et d'une illumination à 660 nm. On constate une production accrue de procollagène 1 induit par un traitement combinant la niacinamide et une illumination par la lumière rouge. Ce couple niacinamide/lumière rouge, pouvant être présent dans le procédé de traitement de l'invention, vient apporter au traitement des peaux grasses un soin supplémentaire pour améliorer l'état général de la peau. C'est en outre le cas de l'effet hydratant (figure 6) du procédé de traitement de l'invention qui s'oppose à la majeure partie des traitements existants pour les peaux à tendance grasse qui, au contraire, ont tendance à assécher la peau.

Ainsi, le procédé de traitement de l'invention, en plus de réduire significativement la production de sébum et le caractère inflammatoire également impliqué dans la production de sébum, fait office de soin en améliorant l'état général de la peau.

Le procédé de traitement de l'invention trouve application non seulement pour le traitement esthétique des peaux à tendance grasse, mais pour tout type de séborrhée en général (dermite séborréique par exemple).

L'invention porte en outre sur un dispositif de mise en œuvre du procédé de l'invention. Le dispositif de l'invention est décrit en référence aux figures 12 et 13.

Le dispositif 30 comprend un boitier 31 de forme générale cylindrique qui comporte un dispositif électroluminescent 32 et une capsule amovible 33 d'application de la composition sur la peau contenant ladite composition. Les parois du dispositif présentent une partie embossée qui relève exclusivement d'un caractère esthétique. Le dispositif électroluminescent 32 présente principalement une série de diodes électroluminescentes (LEDs) 29 réparties régulièrement et circonférentiellement autour de l'axe principal XX' du côté d'une première extrémité 30a du dispositif 30 et montées sur une carte électronique dédiée 34. Chaque LED 29 est surmontée d'une lentille 35 formant guide de lumière et assurant la focalisation de la lumière émise par les LEDs 29 dans une direction contrôlée comme il sera explicité plus loin. Dans un mode de réalisation préférentiel, neuf LEDs 29 sont agencées circonférentiellement sur la carte électronique 34. Les LEDs sont reliées électriquement à une batterie 36 située au niveau du manche du dispositif 30 du côté de son extrémité opposée 30b.

En référence à la figure 13, le dispositif comporte, en amont des LEDs 29 du coté de sa première extrémité 30a un logement 37 de réception de la capsule amovible 33. Ce logement 37 présente une forme générale tronconique mais peut présenter toute forme adaptée coïncidant avec la structure de la paroi externe de la capsule 33. Deux crochets 38 diamétralement opposés sont ménagés à proximité de l'ouverture 39 du logement 37 pour assurer la solidarisation amovible de la capsule 33 insérée dans le logement de réception 37 selon la flèche F.

En référence à la figure 13, la capsule 33 présente principalement un capuchon 40 amovible relativement à ladite capsule 33, un distributeur 41 sous forme de boule, un réservoir de composition 43 relié au distributeur 41 par au moins une ouverture 42 assurant la distribution de la composition sur la surface de la boule 41, et une paroi de fond 44 mobile en coulissement relativement à la boule 41 entre une position la plus éloignée de la boule 41 dans laquelle la composition reste dans le réservoir 43, et une position la plus proche de la boule 41 dans laquelle la composition, ou une partie de la composition, contenu dans le réservoir 43 est libérée sur la surface de la boule 41. Les parois latérales 45 de la capsule 33 sont translucides ou transparentes pour assurer la diffusion de la lumière sur la surface et dans la direction visée. Plus particulièrement, les lentilles 35 associées aux LEDs 29 sont configurées pour assurer la distribution de la lumière émise dans les parois 45 de la capsule elles-mêmes configurées pour faire converger la lumière au niveau de la boule de distribution 41 en illuminant ainsi la zone de la peau sur laquelle est simultanément appliquée la composition. En outre, la boule de distribution 41 est maintenue en place par une collerette 48 formant extension des parois latérales 45 de la capsule, et qui est également translucide ou transparente. Ainsi, lors de l'illumination, la lumière est distribuée au niveau de la boule de distribution 41 comme précédemment indiqué, mais également par la collerette 48.

Lorsque la capsule 33 est insérée dans le logement de réception 37, la paroi de fond 44 vient en appui de contact contre un piston 46 dont le déplacement axial en direction de la capsule 33 est assuré par un moteur 47 relié à la batterie 36. Ce déplacement axial non représenté du piston 46 pousse donc la paroi de fond 44 de la capsule 33 vers la boule 41 selon une course définie correspondant à la distribution d'une dose de composition, ce qui entraîne la libération de la composition contenue dans le réservoir 43 sur la surface de cette boule de distribution 41, cette dernière étant destiné à venir rouler sur la surface de la peau à traiter et ainsi à appliquer la composition sur la peau. A l'utilisation suivante, le piston 46 entraîne de nouveau la paroi de fond 44 en déplacement axial vers la boule de distribution 41 sur une nouvelle course pour délivrer une nouvelle dose de composition. A titre d'exemple, la capsule peut être configurée pour délivrer sept doses de composition correspondant à une semaine de traitement.

Un système de commande non représenté permet de commander l'illumination par les LEDs et l'actionnement du piston et la délivrance de la composition sur la peau. Préférentiellement, l'illumination et la délivrance de la composition sont commandées simultanément.

L'homme du métier saura adapter le dispositif de l'invention selon le procédé de l'invention appliqué. Par exemple pour une composition comportant un extrait d' d'*Epilobium fleischeri* et une illumination notamment à 520nm, les LEDs 29 ou partie des LEDs 29 sont configurées pour émettre à 520nm et la composition contenue dans le réservoir 43 de la capsule 13 comprend l'extrait d'*Epilobium fleischeri.* Pour un procédé de l'invention pour lequel l'illumination est faite selon le programme 2 d'illumination, à 520 nm, 660nm et 440 nm, les LEDs 29 ou partie des LEDs 29 sont configurées pour émettre respectivement à 520 nm, 660nm et 440 nm.

A titre d'exemple, la figure 14 illustre un mode de réalisation du dispositif électroluminescent de l'invention 32 et plus particulièrement de l'assemblage de LEDs 29a,29b,29c sur la carte électronique 34. Les LEDs sont dans cet exemple au nombre de 9 et réparties circonférentiellement sur la carte électronique 34 autour du piston 46 qui traverse la carte électronique 34, étant entendu que la référence 29a correspond aux LEDs émettant à 660nm, la référence 29b correspond aux LEDs émettant à 440nm, la référence 29c correspond aux LEDs émettant à 520nm. Le système de commande non représenté peut activer tout ou partie des LEDs, et ainsi mettre en œuvre soit un procédé selon l'invention impliquant une illumination à 520 nm seulement, soit un procédé selon l'invention impliquant une illumination selon le programme d'illumination 2.

## Revendications

1. Procédé de traitement cosmétique non thérapeutique comprenant au moins les étapes de :
- application sur la peau d'un sujet d'une composition active comprenant au moins un extrait d'*Epilobium fleischeri,* et
- illumination de la peau par au moins une source lumineuse émettant à une longueur d'onde comprise entre 490 et 550 nanomètres, de préférence de 520 nanomètres,
l'illumination étant réalisée avant, simultanément ou après l'application de la composition.

2. Procédé selon la revendication précédente, **caractérisé en ce que** la composition active comprend en outre de l'acide salicylique.

3. Procédé selon l'une quelconque des revendications précédentes, **caractérisé en ce que** la composition active comprend en outre de l'extrait d'artichaut.

4. Procédé selon l'une quelconque des revendications précédentes, **caractérisé en ce que** la composition active comprend en outre de la niacinamide, et **en ce que** l'illumination de la peau est en outre réalisée par une source lumineuse émettant à une longueur d'onde comprise entre 620 et 690 nanomètres, de préférence de 660 nanomètres.

5. Procédé selon l'une quelconque des revendications précédentes, **caractérisé en ce que** l'illumination de la peau est en outre réalisée par une source lumineuse émettant à une longueur d'onde comprise entre 410 et 470 nanomètres, de préférence entre 440 et 450 nanomètres.

6. Procédé selon l'une quelconque des revendications précédentes, **caractérisé en ce que** le pourcentage massique en extrait d'*Epilobium* dans la composition est compris entre 0,1 et 10%.

7. Dispositif de traitement cosmétique non thérapeutique comprenant au moins des moyens d'application sur la peau d'un sujet d'une composition active comprenant au moins un extrait d'*Epilobium fleischeri,* laquelle composition active est contenue dans les moyens d'application, et des moyens d'illumination de la peau par au moins une source lumineuse émettant à une longueur d'onde comprise entre 490 et 550 nanomètres, de préférence de 520 nanomètres,
les moyens d'illumination et d'application de la composition active étant configurés pour que l'illumination puisse être réalisée avant, simultanément ou après l'application de la composition.

8. Dispositif selon la revendication précédente, **caractérisé en ce que** la composition active comprend en outre de la niacinamide, et **en ce que** les moyens d'illumination de la peau comportent en outre une source lumineuse émettant à une longueur d'onde comprise entre 620 et 690 nanomètres, de préférence de 660 nanomètres.

9. Dispositif selon la revendication précédente, **caractérisé en ce que** la composition active comprend en outre de l'acide salicylique et de l'extrait d'artichaut, et **en ce que** les moyens d'illumination de la peau comportent en outre une source lumineuse émettant à une longueur d'onde comprise entre 410 et 470 nanomètres, de préférence entre 440 et 450 nanomètres.

## Patentansprüche

1. Nicht-therapeutisches kosmetisches Behandlungsverfahren, das mindestens die folgenden Schritte umfasst:
- Auftragen einer Wirkstoffzusammensetzung, die mindestens einen Extrakt aus *Epilobium fleischeri* enthält, auf die Haut einer Person, und
- Bestrahlung der Haut mit mindestens einer Lichtquelle, die eine Wellenlänge zwischen 490 und 550 Nanometern, vorzugsweise 520 Nanometern, emittiert, wobei
die Bestrahlung vor, gleichzeitig oder nach dem Auftragen der Zusammensetzung erfolgt.

2. Verfahren nach einem beliebigen der vorhergehenden Ansprüche, **dadurch gekennzeichnet, dass** die Wirkstoffzusammensetzung zusätzlich Salicylsäure enthält.

3. Verfahren nach einem beliebigen der vorstehenden Ansprüche, **dadurch gekennzeichnet, dass** die Wirkstoffzusammensetzung zusätzlich Artischockenextrakt enthält.

4. Verfahren nach einem beliebigen der vorstehenden Ansprüche, **dadurch gekennzeichnet, dass** die Wirkstoffzusammensetzung zusätzlich Niacinamid umfasst und dass die Beleuchtung der Haut zusätzlich durch eine Lichtquelle erfolgt, die bei einer Wellenlänge zwischen 620 und 690 Nanometern, vorzugsweise 660 Nanometern, emittiert.

5. Verfahren nach einem beliebigen der vorstehenden Ansprüche, **dadurch gekennzeichnet, dass** die Beleuchtung der Haut zusätzlich durch eine Lichtquelle erfolgt, die eine Wellenlänge zwischen 410 und 470 Nanometern, vorzugsweise zwischen 440 und 450 Nanometern, emittiert.

6. Verfahren nach einem beliebigen der vorstehenden Ansprüche, **dadurch gekennzeichnet, dass** der Massenanteil an Epilobium-Extrakt in der Zusammensetzung zwischen 0,1 und 10 % liegt.

7. Nicht-therapeutische kosmetische Behandlungsvorrichtung, die mindestens Mittel zum Auftragen einer Wirkstoffzusammensetzung, die mindestens einen Extrakt aus *Epilobium fleischeri* enthält, auf die Haut einer Person umfasst, wobei die Wirkstoffzusammensetzung in den Mitteln zum Auftragen enthalten ist, und Mittel zur Beleuchtung der Haut durch mindestens eine Lichtquelle, die eine Wellenlänge zwischen 490 und 550 Nanometern, vorzugsweise 520 Nanometern, emittiert, wobei die Mittel zur Beleuchtung und zum Auftragen der Wirkstoffzusammensetzung so konfiguriert sind, dass die Beleuchtung vor, gleichzeitig oder nach dem Auftragen der Zusammensetzung erfolgen kann.

8. Vorrichtung nach einem beliebigen der vorhergehenden Ansprüche, **dadurch gekennzeichnet, dass** die Wirkstoffzusammensetzung zusätzlich Niacinamid enthält und dass die Mittel zum Bestrahlen der Haut zusätzlich eine Lichtquelle umfassen, die eine Wellenlänge zwischen 620 und 690 Nanometern, vorzugsweise 660 Nanometern, emittiert.

9. Vorrichtung nach einem beliebigen der vorhergehenden Ansprüche, **dadurch gekennzeichnet, dass** die Wirkstoffzusammensetzung zusätzlich Salicylsäure und Artischockenextrakt enthält und dass die Mittel zur Beleuchtung der Haut außerdem eine Lichtquelle umfassen, die eine Wellenlänge zwischen 410 und 470 Nanometern, vorzugsweise zwischen 440 und 450 Nanometern, emittiert.

## Claims

1. Non-therapeutic cosmetic treatment method comprising at least the steps of:
- applying to the skin of a subject an active composition comprising at least one extract of *Epilobium fleischeri,* and
- illuminating the skin with at least one light source emitting at a wavelength between 490 and 550 nanometres, preferably 520 nanometres,
the illumination being carried out before, simultaneously with or after the application of the composition.

2. Method according to the preceding claim, wherein the active composition further comprises salicylic acid.

3. Method according to any one of the preceding claims, wherein the active composition further comprises artichoke extract.

4. Method according to any one of the preceding claims, wherein the active composition further comprises niacinamide, and in that the illumination of the skin is further carried out by a light source emitting at a wavelength between 620 and 690 nanometres, preferably 660 nanometres.

5. Method according to any one of the preceding claims, wherein the illumination of the skin is further carried out by a light source emitting at a wavelength between 410 and 470 nanometres, preferably between 440 and 450 nanometres.

6. Process according to any one of the preceding claims, wherein the mass percentage of *Epilobium* extract in the composition is between 0.1 and 10%.

7. Non-therapeutic cosmetic treatment device comprising at least means for applying to the skin of a subject an active composition comprising at least one extract of *Epilobium fleischeri,* which active composition is contained in the application means, and means for illuminating the skin with at least one light source emitting at a wavelength between 490 and 550 nanometres, preferably 520 nanometres,
the means for illuminating and applying the active composition being configured so that illumination can be carried out before, simultaneously with or after application of the composition.

8. Device according to the preceding claim, wherein the active composition further comprises niacinamide, and in that the means for illuminating the skin further comprise a light source emitting at a wavelength between 620 and 690 nanometres, preferably 660 nanometres.

9. Device according to the preceding claim, wherein the active composition further comprises salicylic acid and artichoke extract, and in that the skin illumination means further comprise a light source emitting at a wavelength between 410 and 470 nanometres, preferably between 440 and 450 nanometres.
